(19)

**Europäisches Patentamt**
**European Patent Office**
**Office européen des brevets**

(11) **EP 3 983 994 B1**

(12) **EUROPEAN PATENT SPECIFICATION**

(45) Date of publication and mention
of the grant of the patent:
**10.09.2025 Bulletin 2025/37**

(21) Application number: **20732327.0**

(22) Date of filing: **11.06.2020**

(51) International Patent Classification (IPC):
**G06T 7/00** (2017.01)          **A61B 5/00** (2006.01)

(52) Cooperative Patent Classification (CPC):
**G06T 7/0016;** G06T 2207/10016;
**G06T 2207/10104;** G06T 2207/30016

(86) International application number:
**PCT/IB2020/055483**

(87) International publication number:
**WO 2020/250168 (17.12.2020 Gazette 2020/51)**

(54) **A METHOD FOR MEASURING TRACER UPTAKE IN CEREBRAL TISSUE BY ANALYZING IMAGES ACQUIRED BY POSITRON EMISSION TOMOGRAPHY (PET)**

VERFAHREN ZUR MESSUNG DER TRACER-AUFNAHME IN GEHIRNGEWEBE MITTELS ANALYSE VON DURCH POSITRONENEMISSIONSTOMOGRAFIE AUFGENOMMENEN BILDERN

MÉTHODE DE MESURE D'ABSORPTION DE TRACEUR DANS UN TISSU CÉRÉBRAL PAR ANALYSE D'IMAGES ACQUISES PAR TOMOGRAPHIE PAR ÉMISSION DE POSITONS (PET)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.06.2019 IT 201900008997**

(43) Date of publication of application:
**20.04.2022 Bulletin 2022/16**

(73) Proprietor: **Istituto Nazionale di Fisica Nucleare**
**00044 Frascati (RM) (IT)**

(72) Inventor: **CHINCARINI, Andrea**
**16033 Lavagna (Genova) (IT)**

(74) Representative: **Vanzini, Christian et al**
**Jacobacci & Partners S.p.A.**
**Corso Emilia 8**
**10152 Torino (IT)**

(56) References cited:
• **CECCHIN DIEGO ET AL:** "A new integrated dual time-point amyloid PET/MRI data analysis method", EUROPEAN JOURNAL OF NUCLEAR MEDICINE, SPRINGER VERLAG, HEIDELBERG, DE, vol. 44, no. 12, 4 July 2017 (2017-07-04), pages 2060 - 2072, XP036342877, ISSN: 1619-7070, [retrieved on 20170704], DOI: 10.1007/ S00259-017-3750-0
• **ANDREA CHINCARINI ET AL:** "Standardized Uptake Value Ratio-Independent Evaluation of Brain Amyloidosis", JOURNAL OF ALZHEIMER'S DISEASE, vol. 54, no. 4, 18 October 2016 (2016-10-18), NL, pages 1437 - 1457, XP055666022, ISSN: 1387-2877, DOI: 10.3233/JAD-160232
• **A. CHINCARINI ET AL:** "A kinetics-based approach to amyloid PET semi-quantification", EUROPEAN JOURNAL OF NUCLEAR MEDICINE, 25 January 2020 (2020-01-25), DE, XP055665814, ISSN: 1619-7070, DOI: 10.1007/ s00259-020-04689-y

**(Cont. next page)**

• TIEPOLT SOLVEIG ET AL: "Early [18F] florbetaben and [11C]PiB PET images are a surrogate biomarker of neuronal injury in Alzheimer's disease", EUROPEAN JOURNAL OF NUCLEAR MEDICINE, SPRINGER VERLAG, HEIDELBERG, DE, vol. 43, no. 9, 30 March 2016 (2016-03-30), pages 1700 - 1709, XP036000521, ISSN: 1619-7070, [retrieved on 20160330], DOI: 10.1007/S00259-016-3353-1

• SANTIAGO BULLICH ET AL: "Validation of Noninvasive Tracer Kinetic Analysis of 18 F-Florbetaben PET Using a Dual-Time-Window Acquisition Protocol", THE JOURNAL OF NUCLEAR MEDICINE, vol. 59, no. 7, 2 July 2018 (2018-07-02), US, pages 1104 - 1110, XP055666713, ISSN: 0161-5505, DOI: 10.2967/jnumed.117.200964

**Description**

The present invention generally relates to techniques for measuring tracer uptake in cerebral tissue by analyzing images acquired by means of positron emission tomography (PET).

One specific field of application of these techniques relates to the clinical evaluation of cerebral PET with an amyloid tracer.

The in-vivo quantification of the deposition of cerebral β-amyloid by means of positron emission tomography is emerging as a crucial instrument for diagnosing Alzheimer's disease. Correspondingly, the use of cerebral β-amyloid biotracers in clinical trials is becoming increasingly significant.

When reading the PET images, visual evaluation procedures are currently applied that are carried out by expert operators that make use of a binary approach (positive/negative). The visual evaluation is quick and is easy to carry out in the majority of cases. However, there is a significant fraction of cases where this qualitative approach is anything but trivial.

Therefore, more sophisticated approaches have been proposed.

In particular, quantification (or absolute quantification) methods have been developed that are essentially based on the application of mathematical models to the data collected in order to extract numerical estimates for the parameters associated with physiological processes. The main issue with these methods is their general invasiveness and the discomfort for the patient, which makes them unsuitable for a clinical procedure in practice. Furthermore, all the quantification techniques require a long amount of acquisition time (up to 60-90 minutes depending on the tracer), which increases the patient's discomfort and the likelihood of artifacts associated with motion. Furthermore, the models used may lead to errors when evaluating the kinetic parameters.

Another possible approach for extracting significant quantities from PET images involves the use of semi-quantification (or relative quantification) methods. Compared with absolute quantification methods, these methods represent a more streamlined alternative, although they are less accurate.

The semi-quantification methods are based on static images, which consist of a single acquisition (typical duration: approximately 20 minutes) that begins after a relatively long time after the injection (depending on the tracer, but with typical time delays of at least 40 minutes). The static images minimize the amount of time the patient has to stay in the scanner, thereby reducing acquisition and analysis complexity.

The most used and validated method of the semi-quantification methods is the Standardized Uptake Value ratio (SUVr). The SUV is defined as the ratio between the activity concentration in a region of interest (measured using a calibrated PET scanner) and the dose injected with reference to the bodyweight. When calculating the SUV, the average activity or maximum activity in the region of interest is usually taken into account. The SUVr is therefore given from the ratio between the SUV of the anatomical region under examination and a reference SUV. The SUVr standardizes the activity in a region of interest (up to a single voxel) with the average activity in a reference region of interest. The reference region of interest is normally selected from those regions to which the tracer has a minimum specific bond. The SUVr evaluation has proven to be similar to the visual evaluation. The SUVr method comprises a few disadvantages, the most significant of which consists in the fact that the SUVr values calculated may vary significantly depending on several factors, and in particular on the selection of the reference region of interest.

Cecchin et al: "A new integrated dual time-point amyloid PET/MRI data analysis method", European Journal of Nuclear Medicine, vol. 44, no. 12, 4 July 2017 discloses a method according to the preamble of claim 1.

One object of the present invention is to propose a semi-quantification method that makes it possible to accomplish the known methods using an independent approach in order to provide more robust and diversified knowledge of the cases that are more difficult to read.

In light of this object, the invention relates to a method having the features of claim 1. More specifically, the invention relates to a method for measuring tracer uptake in the cerebral tissue of a patient by analyzing cerebral images acquired by means of positron emission tomography, said cerebral images comprising a static early image acquired immediately after the tracer has been injected into the patient, and a static late image acquired with a given time delay with respect to the early image, wherein the method comprises the following steps:

> selecting a region of interest within said early and late images, the region of interest including the brain parenchyma,
> determining a first domain $D_E$ defined by all the voxels of the region of interest of the early image whose intensity is greater than a first given value,
> determining a second domain $D_L$ defined by all the voxels of the region of interest of the late image whose intensity is greater than a second given value,
> calculating average intensities $<I^L>|_{DE}$ and $<I^L>|_{DL}$ in the first domain $D_E$ and in the second domain $D_L$, respectively, of the late image, and
> calculating the ratio of the average intensities $TDr = <I^L>|_{DE} / <I^L>|_{DL}$.

According to one embodiment, the method also comprises spatially standardizing said early and late images before selecting the region of interest.

The standardization aids the positioning of the region of interest and is a necessary step when taking an interest in a spatial analysis.

The method according to the invention makes it possible to measure the uptake of the specialized tracer in highly perfused regions. Since these are also the regions of greater washout due to the blood flow, measuring the intensity in these regions ensures an increase in the diagnostic contrast between positive subjects (greater tracer uptake) and negative subjects (smaller or no uptake). This is particularly important during the interpretive process since the subjects frequently show regions of altered perfusion both due to existing illnesses and due to pharmacological treatments. The altered perfusion in fact constitutes a confounding element since there is no assumption that the uptake observed in the late scan is dominated by the bonded portion of tracer and not by the portion in the flow.

If amyloid tracers are used, the TDr value obtained by analyzing the PET images may be used by medical personnel as an indication of the presence of cerebral amyloids, on the basis of which they may form their opinion and make decisions for which they are competent.

Compared with the SUVr method, the method according to the invention allows for more precise measurements that are stable over time and unaffected by problems regarding segmentation and selecting the regions of interest.

Although described with reference to amyloid tracers, the method according to the invention may generally be applied to cerebral PET scans acquired using non-receptor tracers in general (also called "flow tracers").

Preferred embodiments of the invention are defined in the dependent claims, which are intended to form an integral part of the present description.

Additional features and advantages of the method according to the invention will become clearer with the following detailed description of one embodiment of the invention, given with reference to the attached drawings that are provided purely by way of non-limiting example, in which

Fig. 1 is a diagram showing a method for acquiring PET images;
Fig. 2 is a diagram showing a method for measuring the tracer uptake according to the invention;
Fig. 3 shows an early PET image, that is taken after the injection of the tracer. The areas surrounded by closed lines represent the domain having the greatest flow, $D_E$;
Fig. 4 shows a late PET image, that is taken with a particular time delay after the injection of the tracer. The areas surrounded by darker closed lines represent the domain having the greatest flow, $D_E$, while the areas surrounded by lighter closed lines represent the domain having the greatest uptake, $D_L$;
Fig. 5 shows the uptake domain $D_L$ in two different subjects, an amyloid-negative and an amyloid-positive subject; and

Fig. 6 shows a sagittal, coronal and axial section of an early image sample (top) and late image sample (bottom), which are spatially standardized and aligned in the Montreal Neurological Institute (MNI) space.

As indicated above, the method according to the invention may be applied to cerebral PET scans acquired using non-receptor tracers in general (also called "flow tracers").

In a preferred embodiment, fluorinated tracers are used (marked with 18F) that are selective for the protein amyloid.

The amyloid tracers are used for the differential diagnosis of Alzheimer's disease. The presence of abnormal deposits of amyloid protein agglomerates is in fact the more selective Alzheimer's marker, the diagnosis of which is independent of the symptomatic evidence (see the most recent criteria established by the NIA-AA National Institute on Aging-Alzheimer's Association; Clifford R. Jack et. al. Toward a biological definition of Alzheimer's disease, Alzheimer's & Dementia (2018)).

As of today, three fluorinated tracers are commercially available that are selective for the protein amyloid: 18F-florbetapir (commercial name AMIViD, trademark of the pharmaceutical company Eli-Lilly), 18F-florbetaben (NeuraCeq, by Piramal) and 18F-flutemetamol (Vizamyl, by GE Healthcare).

Further examples of tracers that may be used for the purposes of the present invention are tracers that are selective for the tau protein (for example 18F-flortaucipir).

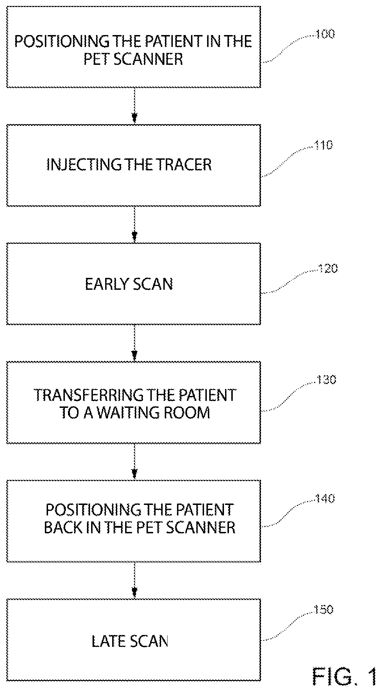

Fig. 1 describes a PET acquisition method that makes it possible to acquire two successive images required for the measurement method according to the invention. First of all, the patient is positioned in the scanner (100) and the tracer is injected (110). An acquisition is therefore carried out within between 0 and 10 minutes after all of the tracer has been injected (early acquisition, step 120). The acquisition may also start 1-2 minutes after injection, depending on the individual protocol and on the injection method. After this, the patient is taken out of the scanner and held in a suitable adjacent waiting room (130). After approximately 30-70 minutes after the injection, for example (the exact time depends on the tracer), the patient is positioned back in the scanner (140) for a second acquisition (late acquisition, step 150) that lasts for approximately 20 minutes (this duration is also dependent on the tracer). After this, the two static images or scans taken at two different times after the injection will be referred to as "early" (also indicated by $E$) and "late" (also indicated by $L$).

The PET images acquired are selected by the medical personnel in accordance with quality standards that are known to an expert in the field. In particular, the images that show an absence of motion artifacts, correct field of view that includes the entire brain parenchyma, and a sufficient signal-to-noise ratio will be selected.

Despite being customary and well understood by a nuclear medicine physician, these qualitative recommendations are in the process of being defined by the competent bodies for amyloid tracers (see, for example, Neurol Sci. 2015 Jun; 36(6):1075-81. doi: 10.1007/s10072-015-2079-3. Recommendations from the Italian Interdisciplinary Working Group (AIMN, AIP, SINDEM) for the utilization of amyloid imaging in clinical practice. Guerra UP, Nobili FM, Padovani A, Perani D, Pupi A, Sorbi S, Trabucchi M.).

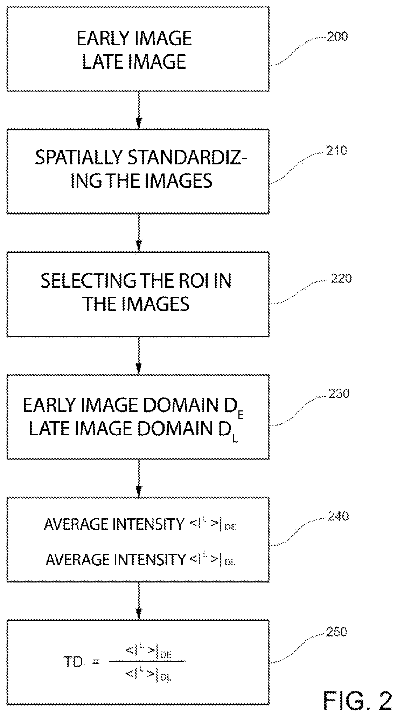

With reference to Fig. 2, the two static cerebral PET images are acquired for an individual for example between 0 and 10 minutes and for example approximately 30 minutes, respectively, from when the non-receptor tracer was administered, as described above (step 200).

First and second images (210) therefore proceed to be spatially standardized. The early image is typically used as the "moving image" and the late image as the "fixed image."

Therefore, a region of interest is selected (brain ROI) that includes the brain parenchyma and excludes the theca, the ventricles, the cerebellum and the brain stem (220).

A region or domain $D_E$ is therefore determined within the context of the first PET image (early image), the voxels of which have a greater intensity value than a first preset level, for example corresponding to the 85% percentile (230). Furthermore, a region or domain $D_L$ is determined within the context of the second PET image (late image), the voxels of which have a greater intensity value than a second preset level, for example corresponding to the 95% percentile.

The average intensities $<I^L>|_{DE}$ and $<I^L>|_{DL}$ are then calculated in the domain $D_E$ and in the domain $D_L$, respectively, of the late image.

Lastly, the ratio of the average intensities is then calculated according to the equation $TDr = <I^L>|_{DE} / <I^L>|_{DL}$.

*Spatial standardization* (step 210)

The spatial standardization step consists in researching a 6-parameter transformation matrix (3 translation and 3 rotation parameters), optimization metrics (Mutual Information) and software for looking for the minimum (for example ANTs, among the numerous ones available) in order to superimpose the early and late scans such that the anatomical regions coincide. This step is necessary, since the patient has been repositioned in the scanner between one scan and the other. Since the images are of the same patient and have been acquired using the same scanner, it is sufficient to use a 6-parameter transformation.

The PET images may optionally be standardized on cerebral models that are processed using magnetic resonance acquisitions and are available in anonymized databases in order to render the presentation and reading of the scans consistent with the anatomical conventions used in medicine. The additional effect would be that of being able to benefit from regionalized information (and therefore originating from a portion of the brain) instead of the sole global value calculated over the entire parenchyma. For this purpose, the additional spatial standardization of the existing templates makes it possible to immediately use numerous segmen-tations (atlases) that are available for clinical research.

*Selecting a region of interest (brain ROI)* (step 220)

This selection is made by means of mapping a suitable pre-segmented mask on the individual patient by means of non-linear transformation.

The pre-segmented mask mentioned above consists of a trace outlined on a three-dimensional "sample" image (available for free) that contains all of the brain, and various structures of which may be included/excluded at will. In order to be able to adapt this mask to the patient in question, it is necessary to take advantage of a spatial standardization method having a similar concept to that described in the "spatial standardization" point, with the difference that the transformation matrix is far more complex. The method is known as a non-linear transformation method and consists in defining a deformation field that moves the "sample" image closer to that of the patient by means of a point-to-point deformation. Even though the transformation is complex, the ingredients and the software to be used are the same as those described in the "spatial standardization" point. Once the deformation field has been found, it is applied to the mask to obtain the equivalent thereof in the patient's space (brain ROI).

This step ensures that the information to be analyzed originates from the regions of clinical interest in the brain parenchyma and not from confounding structures (for example the skull or the neck).

*Determination of $D_E$ and $D_L$* (step 230)

The selection of a large ROI based on anatomical factors, which are specific to each individual, is followed by a study of the statistics of the counts - that is of the intensity of the signal recorded - within the brain ROI itself.

Therefore, the voxels belonging to the brain ROI are selected and a few elementary statistics (quantiles) are calculated. The process may be carried out in principle using any computing software, even if some (for example, MATLAB, R or Python) allow for the process with great ease.

Therefore, the intensity value corresponding to a certain percentile of the distribution of the counts inside the brain ROI is calculated (it is worth remembering that, in this step, the early and late scans are co-located in the same space and therefore the brain ROI is applicable to both).

As indicated above, the region $D_E$ is defined by all the voxels belonging to the brain ROI of the "early" scan, the intensity of which is greater than the value that corresponds to the 85% percentile, for example, calculated over the distribution of the intensities. More generally, the percentile value may be between 75% and 95%; however, preliminary studies suggest an optimum value of around 85%.

As indicated above, the region $D_L$ is defined by all the voxels belonging to the brain ROI of the "late" scan, the intensity of which is greater than the value that corresponds to the 95% percentile, for example, calculated over the distribution of the intensities. More generally, the percentile value may be between 80% and 99%; however, preliminary studies suggest an optimum value of around 95%.

*Calculating the ratio of the average intensities* (steps 230-240)

This ratio is the *TDr* of the subject, or rather the ratio between the intensities calculated using two domains, one of which is defined on the first scan. However, the average intensities are always calculated over the second scan (late). In fact, while the region $D_L$ is already defined on the late scan, the region $D_E$ is defined on the early scan but is applied to the late scan (by virtue of the spatial co-location thereof after the recording).

The acronym of the method appropriately highlights the time delay implicit when applying the region $D_E$ to the late scan. All of the intensities are therefore calculated from the second scan for the two regions, one of which (the $D_E$) has been defined on the first scan.

The method according to the invention, also referred to in the following as the Time-Delayed ratio (TDr) method, is a semi-quantification technique based on the kinetics of the tracer.

The kinetic information is derived from two static images or scans, in particular from an "early" image and from a "late" image.

The early scan is an acquisition that begins just before the injection process, during the injection process or immediately after the injection process (for example after a few minutes, in particular after 1-2 minutes), and lasts for a few minutes, in particular for less than 10 minutes, for example for approximately 5 minutes. The late scan is a standard static scan used to evaluate the amyloid PET (for example carried out 50-70 minutes or 90-110 minutes after the injection process depending on the tracer).

In approximately the first 5 minutes, the signal is dominated by the tracer in the blood flow such that this scan may also be taken as a representation of the perfusion. The TDr method differs from the SUVr method in terms of the definition of the uptake and reference regions of interest. In its simplest form, the *TDr* ratio is defined as *TDr* = $<I^L>|_{DE}$ / $<I^L>|_{DL}$, where $<I^L>|_{DE}$ is the average intensity measured on the late scan and averaged over the domain or region $D_E$, while $<I^L>|_{DL}$ is the average intensity measured on the late scan and averaged over the domain or region $D_L$. The domains are a group of regions of interest defined in the early image and in the late image as follows:

$$D_E = v \in E | I_v \geq I_0^E \qquad (1)$$

$$D_L = v \in L | I_v \geq I_0^L \qquad (2)$$

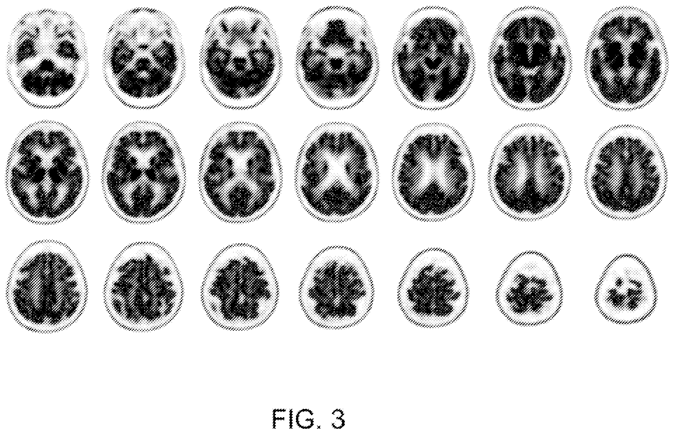

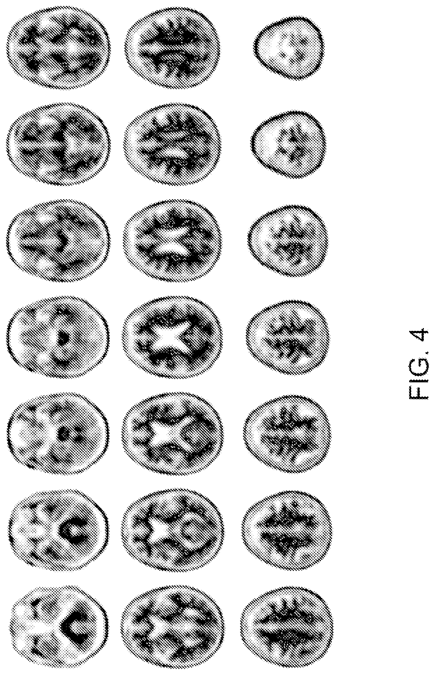

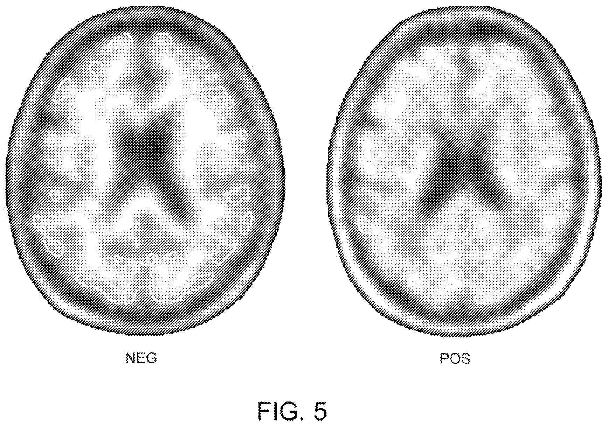

where v represents the voxels in the image and $I_v$ is the intensity *in v*. $D_E$ and $D_L$ are therefore each a group of voxels whose intensity is greater than a given value (see Fig. 3 and 4, for example). Therefore, TDr is an intensity ratio similar to the SUVr but the regions of interest used in the calculation are not predefined or determined by the data. These regions are, instead, adapted to the patient and differ from patient to patient (see Fig. 5, for example) and from image to image, again in the same patient (in the case of a longitudinal study).

The TDr method takes inspiration from the absolute quantification approach. The tracer initially flows in the blood flow and therefore slowly migrates into the tissues. It is therefore possible to select tissues having a high flow (limiting the research to the cortical areas) from the early image. If β-amyloid is present in these tissues, the tracer will bind to said tissues and we have the advantage of knowing that it is appropriately distributed and in a relatively large amount (high uptake). In contrast, if β-amyloid is absent, the tracer will almost certainly be washed out since the tissue is well perfused (effective removal).

The numerator in the TDr ratio therefore describes the intensity that is obtained in the late scan averaged over the voxels having a high blood flow defined in the early scan. As a result, a high $<I^L>|_{DE}$ value corresponds to a high uptake, and vice versa.

Now, given that the absolute value for this intensity may vary between individuals for a variety of reasons (injected activity, blood flow, physiological characteristics, etc.), this intensity must be standardized. The denominator in the TDr ratio describes the intensity that is obtained in the late image on the voxels having a high uptake (specific or non-specific) that are defined in the same late scan. Therefore, instead of using a fixed reference tissue (such as the cerebellum), the domain having the greatest uptake is used, otherwise known as the hot spot, which may be identified in the gray matter or in the adjacent white matter.

This standardization to some extent frees the TDr from the constraints of accuracy of capturing the images and of defining a reference tissue. Another immediate benefit consists in the fact that $D_E$ and $D_L$ are defined on images that are acquired using the same scanner and are reconstructed using the same method. This means that the partial-volume effect (PVE) - although still present - is partially "compensated for" by the selection of the two domains in the same space. Due to this definition and to the selection of regions having a high flow, the measurement of the amyloid burden in these areas is more accurate since the contrast between the burden and the hot spot is greatest.

The domains $D_E$ and $D_L$ are determined by the features of the patient and reference regions or cortical atlases are not required. The domains typically consist of different separate regions of interest that provide local and global information. Therefore, similarly to the values obtained using the SUVr method, they may be combined or averaged in order to provide regional or consolidated information.

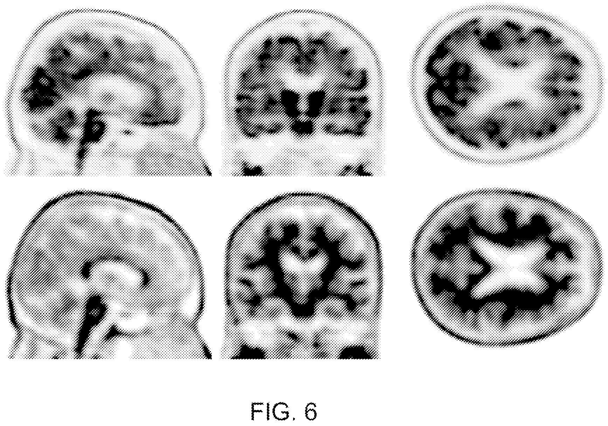

The TDr only requires basic images to be processed, consisting of a spatial recording with respect to the coordinates of a normative space, such as the reference of the Montreal Neurological Institute (MNI). The brain does not need to be segmented from the head. The only unique requirement is that the early scan (E) is recorded on the late scan (L) in the native space before every other spatial or intensity standardization. This makes it possible to minimize spatial resampling errors (Fig. 6).

Once the scans E and L have been aligned in the native space, the scan L guides the transformation to place the scans in the normative space (for example, MNI).

The implementation of the TDr method is mainly linear. It only requires an accurate recording of the early scan on the late scan such that the regions of interest of the domain $D_E$ may be correctly applied thereto; this is possible with a 6-parameter linear transformation, with the scan L as the fixed image. All the subsequent transformations (that is on the normative space) are applied to both the images E and L.

After appropriate registration of the images and once in the MNI space, a mask is applied to cut the scalp, brainstem, cerebellum and basal ganglia and to estimate the higher counts according to equations (1) and (2), in which, for example, $I_0^E = 0.85$ percentile and $I_0^L = 0.99$ percentile.

The use of specific regions of interest of the patient allows for more precise measurements, thereby allowing for greater reproducibility and therefore application to longitudinal studies, or rather the possibility of monitoring the response of the patient to anti-amyloid therapies, currently in phase III of the trial.

One variant of the analysis method proposed provides that the domain $D_L$ is used together with an anatomical atlas to identify subregions of interest. In this way, it is possible to obtain more complex information that may allow for the identification of specific accumulation patterns and therefore the typification of the patient to the advantage of the diagnostics and the response to the drugs.

The method proposed is already provided for this type of analysis, thus only requiring further spatial standardization as described in the second paragraph of the "spatial standardization" point.

The selection of the atlas for partitioning the cerebral subregions does not fall within the scope of the present method; in fact, there are several possibilities selected that are generally dictated by clinical opportunity reasons. However, the TDr method does not have any inherent limitations and was tested on a large variety of atlases that are reasonably adapted to the anatomy and have clinical significance. In fact, the only connection is that the intersection of the domain $D_L$ with any other region of the atlas is not zero.

## Claims

1. A computer-implemented method for measuring tracer uptake into cerebral tissue of a patient by analysis of cerebral images acquired by positron emission tomography, said cerebral images comprising a static early image acquired from the injection of the tracer into the patient, and a static late image acquired with a given time delay with respect to the early image, wherein the method is **characterized by** comprising the following steps:

   selecting a region of interest within said early and late images, the region of interest including the brain parenchyma,
   determining a first domain $D_E$ defined by all voxels of the region of interest of the early image whose intensity is greater than a first given value,
   determining a second domain $D_L$ defined by all voxels of the region of interest of the late image whose intensity is greater than a second given value,
   calculating average intensities $<I^L>|_{DE}$ and $<I^L>I_{DL}$ in the first domain $D_E$ and in the second domain $D_L$, respectively, on the late image,
   calculating the ratio of the average intensities $TDr = <I^L>|_{DE} / <I^L>I_{DL}$.

2. A method according to claim 1, further comprising spatially normalizing said early and late images.

3. A method according to claim 1 or 2, wherein the early image is acquired within about 10 minutes from injection of the tracer.

4. A method according to any of the preceding claims, wherein the late image is acquired after at least about 30 minutes from injection of the tracer.

5. A method according to any of the preceding claims, wherein said first value is comprised between 75% and 95% percentile, and said second value is comprised between 80% and 99% percentile.

## Patentansprüche

1. Computerimplementiertes Verfahren zur Messung der Tracer-Aufnahme in Gehirngewebe eines Patienten mittels Analyse von durch Positronenemissionstomographie aufgenommenen Gehirnbildern, wobei die Gehirnbilder ein statisches Anfangsbild umfassen, das nach der Injektion des Tracers in den Patienten aufgenommen wurde, und ein statisches Letztbild, das mit einer bestimmten zeitlichen Verzögerung in Bezug auf das Anfangsbild aufgenommen wurde, wobei das Verfahren **dadurch gekennzeichnet ist, dass** es die folgenden Schritte umfasst:

   Auswählen eines Interessensbereichs innerhalb der Anfangs- und Letztbilder, wobei der Interessensbereich das Hirnparenchym umfasst,
   Bestimmen eines ersten Bereichs $D_E$, der durch alle Voxels des Interessensbereichs des Anfangsbilds definiert wird, deren Intensität größer als ein erster vorgegebener Wert ist,
   Bestimmen eines zweiten Bereichs $D_L$, der durch alle Voxels des Interessensbereichs des Letztbilds definiert wird, deren Intensität größer als ein zweiter vorgegebener Wert ist,
   Berechnen der mittleren Intensitäten $<I^L>|_{DE}$ und $<I^L>|_{DL}$ in dem ersten Bereich $D_E$ und in dem zweiten Bereich $D_L$ jeweils, in dem Letztbild,
   Berechnen des Verhältnisses der mittleren Intensitäten TDr = . $<I^L>|_{DE}$ und $<I^L>|_{DL}$

2. Verfahren nach Anspruch 1, ferner umfassend Normalisieren räumlich der Anfangs- und Letztbilder.

3. Verfahren nach Anspruch 1 oder 2, wobei das Anfangsbild innerhalb von etwa 10 Minuten nach der Injektion des Tracers aufgenommen wird.

4. Verfahren nach einem der vorhergehenden Ansprüche, wobei das Letztbild nach mindestens etwa 30 Minuten nach der Injektion des Tracers aufgenommen wird.

5. Verfahren nach einem der vorhergehenden Ansprüche, wobei der erste Wert zwischen dem 75. und 95. Perzentil liegt und der zweite Wert zwischen dem 80. und 99. Perzentil liegt.

## Revendications

1. Procédé mis en œuvre par ordinateur pour mesurer l'absorption d'un traceur dans un tissu cérébral d'un patient par analyse d'images cérébrales acquises par tomographie par émission de positons, lesdites images cérébrales comprenant une image statique précoce acquise à partir de l'injection du traceur dans le patient, et une image statique tardive acquise avec un certain délai temporel par rapport à l'image précoce, ledit procédé étant **caractérisé en ce qu'**il comprend les étapes suivantes :

   sélection d'une région d'intérêt au sein desdites images précoce et tardive, la région d'intérêt incluant le parenchyme cérébral,
   détermination d'un premier domaine $D_E$ défini par tous les voxels de la région d'intérêt de l'image précoce dont l'intensité est supérieure à une première valeur donnée,
   détermination d'un second domaine $D_L$ défini par tous les voxels de la région d'intérêt de l'image tardive dont l'intensité est supérieure à une seconde valeur donnée,
   calcul des intensités moyennes $<I^L>|_{DE}$ et $<I^L>|_{DL}$ dans le premier domaine $D_E$ et dans le second domaine $D_L$, respectivement, sur l'image tardive,
   Calcul du rapport des intensités moyennes TDr = $<I^L>|_{DE}/<I^L>|_{DL}$.

2. Procédé selon la revendication 1, comprenant en outre
   la normalisation spatiale desdites images précoce et tardive.

3. Procédé selon la revendication 1 ou 2, dans lequel l'image précoce est acquise dans un délai d'environ 10 minutes à compter de l'injection du traceur.

4. Procédé selon l'une quelconque des revendications précédentes, dans lequel l'image tardive est acquise après au moins environ 30 minutes à compter de l'injection du traceur.

5. Procédé selon l'une quelconque des revendications précédentes, dans lequel ladite première valeur est com-prise entre le 75% et le 95%, et ladite seconde valeur est comprise entre le 80% et le 99%.

FIG. 1

EARLY IMAGE
LATE IMAGE — 200

SPATIALLY STANDARDIZ-
ING THE IMAGES — 210

SELECTING THE ROI IN
THE IMAGES — 220

EARLY IMAGE DOMAIN $D_E$
LATE IMAGE DOMAIN $D_L$ — 230

AVERAGE INTENSITY $<I^k>|_{D_E}$

AVERAGE INTENSITY $<I^k>|_{D_L}$ — 240

$$TD = \frac{<I^k>|_{D_E}}{<I^k>|_{D_L}}$$ — 250

FIG. 2

FIG. 3

FIG. 4

NEG                    POS

FIG. 5

FIG. 6

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CECCHIN et al.** A new integrated dual time-point amyloid PET/MRI data analysis method. *European Journal of Nuclear Medicine*, 04 July 2017, vol. 44 (12)

- **CLIFFORD R.** Toward a biological definition of Alzheimer's disease, Alzheimer's & Dementia. NIA-AA National Institute on Aging-Alzheimer's Association, 2018
- *Neurol Sci.*, June 2015, vol. 36 (6), 1075-81